# EUROPEAN PATENT SPECIFICATION

(11) **EP 4 189 376 B1**
(45) Date of publication and mention of the grant of the patent: **29.10.2025**
(21) Application number: 21745833.0
(22) Date of filing: 29.07.2021
(51) Int. Cl.: G01N 27/416, G01N 27/49

(54) **ELECTROCHEMICAL SENSOR SYSTEMS FOR SENSING ANALYTICAL REACTIONS AND BIOLOGICAL OPERATIONS AND METHODS**
ELEKTROCHEMISCHE SENSORSYSTEME ZUR ERFASSUNG ANALYTISCHER REAKTIONEN UND BIOLOGISCHER VORGÄNGE UND VERFAHREN
SYSTÈMES DE CAPTEURS ÉLECTROCHIMIQUES POUR LA DÉTECTION DES RÉACTIONS ANALYTIQUES ET DES OPÉRATIONS ET PROCÉDÉS BIOLOGIQUES

(30) Priority: 30.07.2020 EP 20382702
(43) Date of publication of application: 07.06.2023
(73) Proprietor: Fundació Institut de Bioenginyeria de Catalunya, 08028 Barcelona (ES); Universitat de Barcelona, 08007 Barcelona (ES); Consorcio Centro de Investigación Biomédica en Red, 28029 Madrid (ES)
(72) Inventor: SAMITIER MARTÍ, Josep, 08028 BARCELONA (ES); MIR LLORENTE, Mònica, 08028 BARCELONA (ES); DULAY BACENA, Samuel, 08028 BARCELONA (ES); RIVAS TORCATES, Lourdes Josefina, 08028 BARCELONA (ES); MISERERE, Sandrine, 08028 BARCELONA (ES); PÉREZ LÓPEZ, Briza, 08028 BARCELONA (ES); LOPEZ DE MIGUEL, Manuel, 08028 BARCELONA (ES); PALACIO BONET, Francisco, 08028 BARCELONA (ES)
(74) Representative: ZBM Patents - Zea, Barlocci & Markvardsen
(86) International application number: PCT/EP2021/071340
(87) International publication number: WO 2022/023499

(56) References cited:
- WO-A1-01/33207
- US-A- 5 523 692
- DARYL MA ET AL: "Concurrent Potentiometric and Amperometric Sensing with Shared Reference Electrodes", IEEE SENSORS JOURNAL, 1 January 2020 (2020-01-01), USA, pages 1 - 1, XP055763276, ISSN: 1530-437X, DOI: 10.1109/JSEN.2020.3039567
- "State of the Art in Biosensors - General Aspects", 13 March 2013, INTECH, ISBN: 978-953-51-1004-0, article JAIME PUNTER ET AL: "Bioelectronics for Amperometric Biosensors", XP055533156, DOI: 10.5772/52248
- HAITAO LI ET AL: "CMOS Electrochemical Instrumentation for Biosensor Microsystems: A Review", SENSORS, vol. 17, no. 12, 31 December 2016 (2016-12-31), pages 74, XP055574869, DOI: 10.3390/s17010074
- BUSONI L ET AL: "A comparison between potentiostatic circuits with grounded work or auxiliary electrode", REVIEW OF SCIENTIFIC INSTRUMENTS, AIP, MELVILLE, NY, US, vol. 73, no. 4, 1 April 2002 (2002-04-01), pages 1921 - 1923, XP012039988, ISSN: 0034-6748, DOI: 10.1063/1.1463715
- SCHELTER W ET AL: "COMBINATION OF AMPEROMETRIC AND POTENTIOMETRIC SENSOR PRINCIPLES FOR ON-LINE BLOOD MONITORING", SENSORS AND ACTUATORS B: CHEMICAL, ELSEVIER BV, NL, vol. 6, no. 1-03, 1 January 1992 (1992-01-01), pages 91 - 95, XP009009354, ISSN: 0925-4005, DOI: 10.1016/0925-4005(92)80037-X

## Description

This application claims the benefit of European Patent Application 20382702.7 filed July 30, 2020.

The present invention relates to electrochemical sensor systems for sensing analytical reactions and biological operations. The present invention further relates to methods for sensing analytical reactions and biological operations.

### BACKGROUND

Electrochemical sensors systems using amperometric and potentiometric sensors are generally known.

There are basically two types of amperometric and potentiometric sensors, those that employ two electrodes, and those that employ three electrodes. Two-electrode sensors employ a working electrode and a reference electrode. Three-electrode sensors employ a working electrode, a reference electrode and a counter electrode.

In potentiometric sensors with two electrodes, a potential is measured between the working electrode and the reference electrode. This potential may be proportional to the concentration or the activity of the species to be detected in a measuring medium.

In amperometric sensors with two electrodes, a current is measured. This current is the result of an electroactive substance losing (oxidation) or gaining (reduction) one or more electrons on the surface of the working electrode, wherein the working electrode is under a constant working potential with respect to the reference electrode. The current is proportional to the concentration or the activity of the species to be detected in a measuring medium.

However, there are interferences or cross-talks between amperometric techniques and potentiometric techniques in electrochemical sensors systems comprising amperometric and potentiometric sensors, particularly when the measurements of the amperometric sensors and the potentiometric sensors are performed substantially simultaneously and relatively close to each other.

Examples of the present disclosure seek to at least partially reduce one or more of the aforementioned problems.

Document "Bioelectronics for Amperometric Biosensor", Jaime Punter et al, discloses an amperometric sensor with three electrodes: a working electrode, a counter electrode, and a reference electrode.

Document "CMOS Electrochemical Instrumentation for Biosensors Microsystems : A review" Haitao LI et al, discloses tree-electrode potentiostats.

Document "A comparison between potentiostatic circuits with grounded work or auxiliary electrode" Busoni et al, discloses tree-electrode potentiostats.

US 5 523 692 A discloses an oil deterioration detector comprising a sensitive electrode whose electric potential varies in response to acidity and/or basicity of oil to be measured, and a reference electrode associated with this sensitive electrode.

Document "Combination of amperometric and potentiometric sensor principles for on-line blood monitoring" Schelter W et al, discloses a potentiostats with three-electrodes: a working electrode, a counter electrode, and a reference electrode WO 01/33207 A1 discloses a recording device having at least one potentiometric electrode, at least one amperometric electrode.

Document "Concurrent Potentiometric and Amperometric Sensing with Shared Reference Electrodes", Daryl Ma et al, discloses a method for simultaneously operating potentiometric and amperometric sensors within a single electrochemical cell using a shared reference electrode, achieving high signal correlation and minimal interference.

### SUMMARY

In a first aspect of the invention, an electrochemical sensor system for sensing analytical reactions and biological operations is provided. The electrochemical sensor system comprises: an amperometric sensor comprising two electrodes, wherein the electrodes comprise a working electrode and a reference electrode, wherein the amperometric sensor includes no other electrodes and a potentiometric sensor comprising two electrodes, wherein the electrodes comprise a working electrode and a reference electrode, wherein the potentiometric sensor includes no other electrodes, wherein the reference electrode of the potentiometric sensor is electrically coupled to ground, wherein the working electrode of the amperometric sensor is electrically coupled to the reference electrode of the potentiometric sensor, wherein the amperometric sensor is configured to receive an excitation voltage between the working electrode of the amperometric sensor and the reference electrode of the amperometric sensor such that the received excitation voltage is used as a reference voltage of the potentiometric sensor via the reference electrode of the potentiometric sensor.

According to this first aspect, an amperometric sensor and a potentiometric sensor, each of them with two electrodes, are provided. This way, sensors with relatively small size and cost are used. Particularly, in such sensors with two electrodes, the use of a counter electrode is avoided. The counter electrode is usually made with platinum and it may comprise a relatively large diameter of e.g. 125 µm. Such use may imply an extra cost and an extra weight in the electrochemical sensor system.

In summary, the use of an electromechanical sensor system comprising amperometric and potentiometric sensors with only two electrodes facilitates a more compact and cost-effective electromechanical sensor system solution as opposed to electrochemical sensor system solutions involving the use of amperometric and potentiometric sensors with three electrodes.

Moreover, the working electrode of the amperometric sensor is electrically coupled to the reference electrode of the potentiometric sensor such that, in use, an excitation voltage provided between the working electrode of the amperometric sensor and the reference electrode of the amperometric sensor is used as a reference voltage of the potentiometric sensor (via the reference electrode of the potentiometric sensor). With such an arrangement, and by connecting to ground the reference electrode of the potentiometric sensor, a ground or virtual zero is introduced in the working electrode of the amperometric sensor.

In use, when an excitation voltage is provided between the working electrode and the reference electrode of the amperometric sensor, current is established at the working electrode of the amperometric sensor. This current corresponds to a voltage drop caused by the excitation voltage of the amperometric sensor (and the ground or virtual zero introduced by the reference electrode of the potentiometric sensor, which is coupled to ground). Such voltage drop, corresponding to the excitation voltage between the working electrode and the reference electrode of the amperometric sensor, will be used as the reference voltage, via the reference electrode of the potentiometric sensor, by the potentiometric sensor. As a result, interferences between the measurements of the amperometric sensor and the potentiometric sensor are avoided. Additionally, it is possible to perform amperometric and potentiometric measurements substantially simultaneously.

In a second aspect of the invention, a method for sensing analytical reactions and biological operations executable by a control module, is provided. The method comprises: A filtered offset potentiometric voltage from the potentiometric sensor circuit is received. Substantially simultaneously the output amperometric voltage signal from the amperometric sensor circuit is also received. Then, a current measurement related to the received output amperometric sensor voltage from the amperometric sensor circuit and a voltage measurement related to the received filtered offset potentiometric voltage from the potentiometric sensor circuit are determined substantially simultaneously.

According to this second aspect, a first voltage (from the amperometric sensor) and a second voltage (from the potentiometric sensor circuit) are received substantially simultaneously by the control module. Such control module may determine also substantially simultaneously a current measurement related to the received output amperometric sensor voltage from the amperometric sensor circuit and a voltage measurement related to the received filtered offset potentiometric voltage from the potentiometric sensor circuit. It is thus clear that measurements from the amperometric sensor and the potentiometric sensor can be performed (virtually) at the same time and, all this, without interferences or cross talks between such measurements.

### BRIEF DESCRIPTION OF THE DRAWINGS

Non-limiting examples of the present disclosure will be described in the following, with reference to the appended drawings, in which:
Fig. 1 illustrates an example of an excitation circuit; and
Fig. 2a illustrates an example of an amperometric sensor circuit of an amperometric sensor, wherein the amperometric sensor circuit may be coupled to an excitation circuit which may be the same or similar to the one shown in Fig. 1;
Fig. 2b illustrates an example of gain resistor circuit for a transimpedance amplifier (TIA) forming part of the amperometric sensor circuit of Fig. 2a;
Fig. 2c schematically illustrates an example of a reference electrode circuit of the amperometric sensor;
Fig. 3 illustrates an example of a potentiometric sensor circuit of a potentiometric sensor, according to an example;
Fig. 4 illustrates a block diagram of control module comprising an electrochemical sensor system, according to some examples.

### DESCRIPTION OF EMBODIMENTS

Throughout the present disclosure the term *"electrochemical sensor system for sensing analytical reactions and biological operations"* encompasses e.g. operations for the detection of ischemia and hypoxia and, in general, measurements related to the oxygen values and pH values.

Throughout the present disclosure the term *"the amperometric sensor includes no other electrodes"* refers to an amperometric sensor comprising only two electrodes. Similarly, the term *"the potentiometric sensor includes no other electrodes"* refers to a potentiometric sensor comprising only two electrodes.

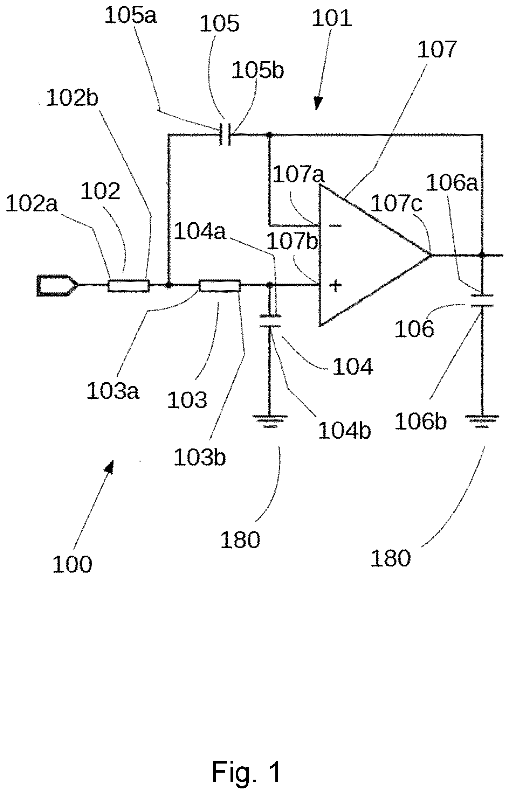

Fig. 1 schematically illustrates an example of an excitation circuit 100. The excitation circuit is configured to provide a variation of voltage as a function of time between the working electrode and the reference electrode of an amperometric sensor. As a result, a current may appear between such electrodes, particularly at the working electrode. This current may be measured using the amperometric sensor, as will be described later on.

The circuit 100 may comprise a low pass filter 101. The low pass filter 101 may comprise a resistor 102 which may have two terminals 102a and 102b, a resistor 103 which may have two terminals 103a, 103b, a capacitor 104 that may have two terminals 104a, 104b, a capacitor 105 which may have two terminals 105a, 105b and a capacitor 106 which may have two terminals 106a, 106b, and an operational amplifier 107. The operational amplifier 107 comprises a negative input terminal 107a, a positive input terminal 107b and an output 107c.

Terminal 102b of the resistor 102 may be coupled to the terminal 105a of the capacitor 105. Terminal 102b of the resistor 102 may further be coupled to the terminal 103a of the resistor 103. Terminal 103b of the resistor 103 may be coupled to the positive input terminal 107b of the amplifier 107. Terminal 103b of the resistor may further be coupled to terminal 104a of the capacitor. Terminal 104b of the capacitor may be coupled to ground 180.

Following the example, terminal 105b of the capacitor 105 may be coupled to the negative input terminal 107a of the operational amplifier 107 and it may be coupled to the output 107c of the operational amplifier 107. The output 107c of the operational amplifier may be coupled to the terminal 106a of the capacitor. The terminal 106b of the capacitor 106 may be coupled to ground 180.

Terminal 102a of the resistor 102 may be coupled to a pulse width modulator (PWM) e.g. a microcontroller (not shown). The microcontroller e.g. a PIC32MX microcontroller provides a pulse-width-modulated (PWM) output. The microcontroller is configured to provide a PWM duty cycle to the PWM output such that a desired voltage may be provided to the terminal 102a of the resistor 102. The above-commented low pass filter 101 is configured to remove alternating signals (pulses) and / or noise of the voltage provided by the microcontroller such that a noiseless voltage (i.e. a voltage with reduced noise) is provided to an amperometric sensor circuit between the working electrode and the reference electrode of such amperometric circuit, as will be explained later on.

It is noted that the operational amplifier 107 may be configured to operate either as an inverter amplifier for providing a negative voltage to the amperometric circuit or as a voltage follower such that a positive voltage is provided to the amperometric circuit (particularly between the working electrode and the reference electrode). The voltage provided to the amperometric circuit may be between -3 volts and +3 volts.

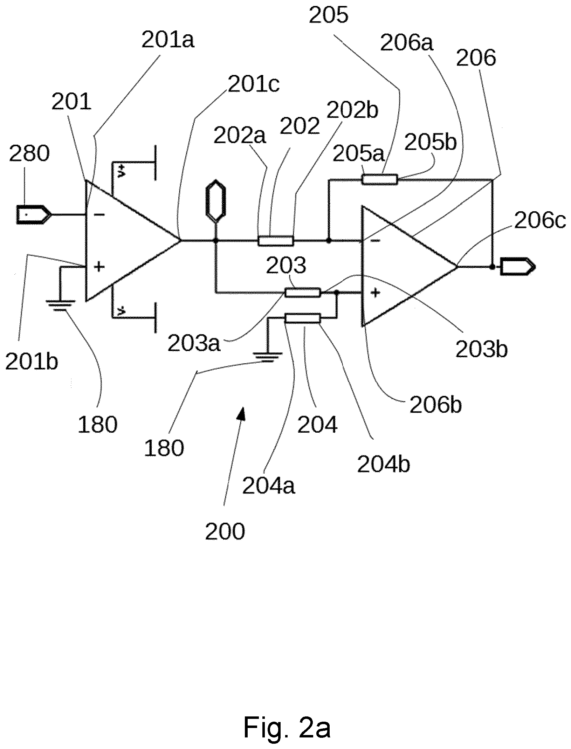

Fig. 2a illustrates an example of an amperometric sensor circuit which may be coupled to an excitation circuit which may be the same or similar to the one shown in Fig. 1. The amperometric sensor circuit forms part of an amperometric sensor. The amperometric sensor circuit 200 may comprise a transimpedance amplifier (TIA) 201 including a negative input terminal 201a, a positive input terminal 201b and an output 201c, a resistor 202 including two terminals 202a, 202b, a resistor 203 including two terminals 203a, 203b, a resistor 204 including two terminals 204a, 204b, a gain resistor 205 including two terminals 205a, 205b and an operational amplifier 206. The operational amplifier 206 comprises a negative input terminal 206a, a positive input terminal 206b and an output 206c.

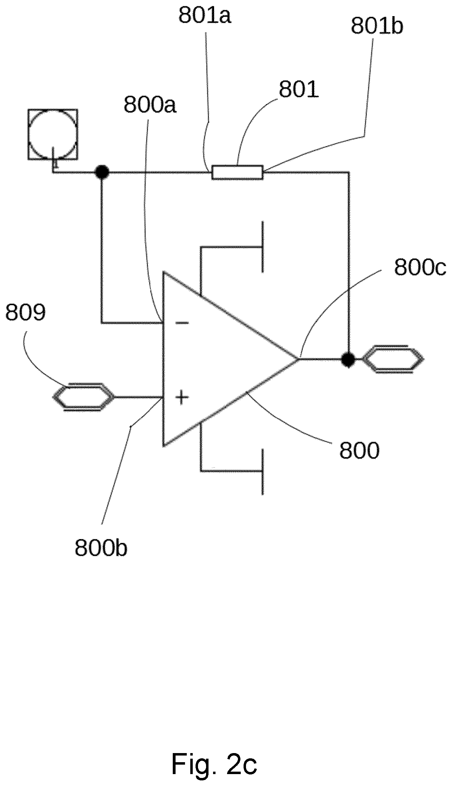

The negative input terminal 201a of the TIA 201 may be electrically coupled to a working electrode 280 of the amperometric sensor. The positive input terminal of the TIA 201 may be coupled to ground 180. An excitation voltage may be applied between the working electrode 280 and a reference electrode (not shown in this figure) by an excitation voltage circuit as hereinbefore described. As a result, current appears at the working electrode. Such current is sensed by the working electrode 280. It is noted that, as commented above, the circuit of the reference electrode forming part of the amperometric sensor is not shown in this figure. This circuit will be described later on with reference to Fig. 2c

In any case, the current which appears at the working electrode corresponds to the voltage drop caused by the excitation voltage provided by the excitation voltage circuit and the ground 180 or virtual zero introduced by a reference electrode (which is coupled to ground) of a potentiometric sensor, as will be described later on. In this respect, the reference electrode of the potentiometric sensor is coupled to the working electrode of the amperometric sensor. It is noted that this voltage drop i.e. the excitation voltage provided between the working electrode and the reference electrode of the amperometric sensor will be used as the reference voltage (sensed by the reference electrode) for the potentiometric sensor, as also will be described later on.

The TIA 201 may be configured to convert the current sensed by the working electrode 280 of the amperometric sensor to a proportional output voltage.

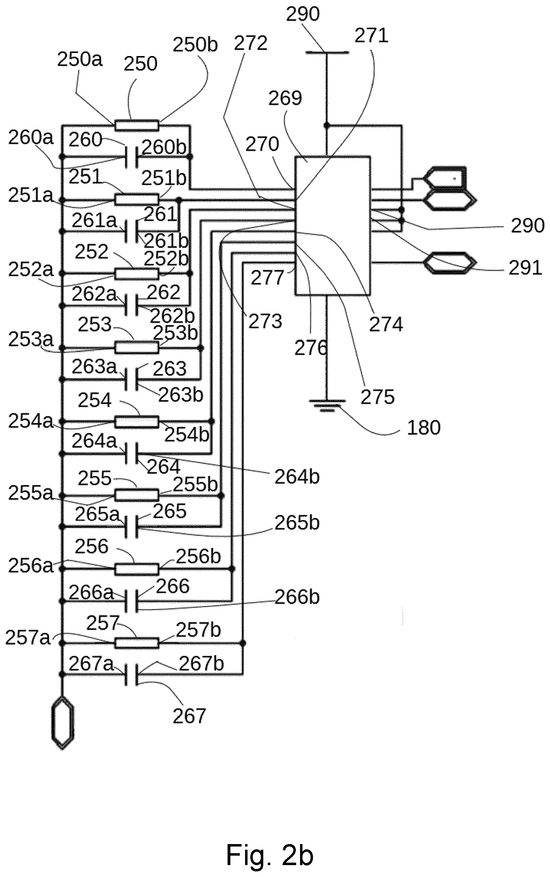

Following the example, a gain resistor (not shown) may be coupled between the negative terminal of the TIA 201 and the output 201c of the TIA 201. As shown in Fig. 2b, a circuit configured to select a gain resistor of the TIA is provided. The circuit may comprise eight resistors 250 - 257 wherein each resistor comprises two terminals 250a - 257a, 250b - 257b. The circuit further comprises eight capacitors 260 - 267 wherein each capacitor comprises two terminal 260a - 267a, 260b - 267b.

The circuit further comprises a CMOS analog matrix switch 269. The switch comprises eight inputs 270 - 277. The terminal 250b of the resistor 250 and the terminal 260b of the capacitor 260 are coupled to the input 270 of the switch. The terminal 251b of the resistor 251 and the terminal 261b of the capacitor 261 are coupled to the input 271 of the switch. The terminal 252b of the resistor 252 and the terminal 262b of the capacitor 262 are coupled to the input 272 of the switch. The terminal 253b of the resistor 253 and the terminal 263b of the capacitor 263 are coupled to the input 273 of the switch. The terminal 254b of the resistor 254 and the terminal 264b of the capacitor 264 are coupled to the input 274 of the switch. The terminal 255b of the resistor 255 and the terminal 265b of the capacitor 265 are coupled to the input 275 of the switch. The terminal 256b of the resistor 256 and the terminal 266b of the capacitor 266 are coupled to the input 276 of the switch. The terminal 257b of the resistor 257 and the terminal 267b of the capacitor 267 are coupled to the input 277 of the switch.

The resistor 250 - 257 may be selected in the range between 1 KOhm and 33 MOhms. The capacitors 260 - 267 are further selected such that non-desired oscillations are avoided.

The switch 269 may comprise a power supply input 290 which may be operated from a power supply of e.g. 2.7 volts to 5.5 volts, specifically 3.3 volts. Such power supply may also be connected to the address inputs 290, 291. The switch may further comprise a ground reference 180 coupled to ground. The switch may also comprise a *"serial clock line"* input which may be used in conjunction with a *"serial data line"* input to clock data into the 8-bit input shift register.

Again in Fig. 2a, the output 201c of the TIA 201 may further be coupled to the terminal 202a of the resistor 202 and the terminal 203a of the resistor 203. The terminal 202b of the resistor 202 may be coupled to the input negative terminal 206a of the operational amplifier 206. The output terminal 203b of the resistor 203 may be coupled to the input positive terminal 206b of the operational amplifier 206. Additionally, the resistor 204 may be connected to ground 180 via the terminal 204a and to the input positive terminal 206b of the TIA via the terminal 204b.

The terminal 205a of the gain resistor 205 may be connected to the negative input terminal 206a of the operational amplifier and the terminal 205b may be connected to the output 206c of the operation amplifier 206.

The operational amplifier 206 may be configured to operate as an inverter amplifier if the proportional voltage outputted by the transimpedance amplifier 201 is a negative voltage such that the negative voltage is converted to a positive voltage. In some other examples, if the proportional voltage outputted by the transimpedance amplifier 201 is a positive voltage, the operational amplifier 206 may be configured to operate as a follower amplifier. The reasoning of such an arrangement is that a control module including e.g. an AC-DC converter (not shown in this figure) may be coupled to the output terminal of the operational amplifier 206, as will be explained later on. The AC-DC converter may be configured to operate in a voltage range between 0 volts and 3.3 volts and thus, if a negative voltage is outputted by the operational amplifier, such negative voltage may not be detected by the AC-DC converter. In any case, the gain of the operational amplifier 206 may be one.

Fig. 2c shows an example of a reference electrode circuit of the amperometric sensor. The reference electrode circuit of the amperometric sensor may comprise an operational amplifier 800 comprising a negative input terminal 800a, positive input terminal 800b and an output terminal 800c. The circuit further comprises a resistor 801 with two terminals 801a, 801b.

The reference electrode 809 of the amperometric sensor may be connected to the positive input terminal 800b of the operational amplifier 800. The terminal 801a of the resistor 800 may be connected to the negative input terminal 800a of the operational amplifier 800. The terminal 801b of the resistor may be connected to the output 800c of the operational amplifier 800. In examples, the resistor 801 may have zero ohms. The operational amplifier may be configured to act as a potentiostat.

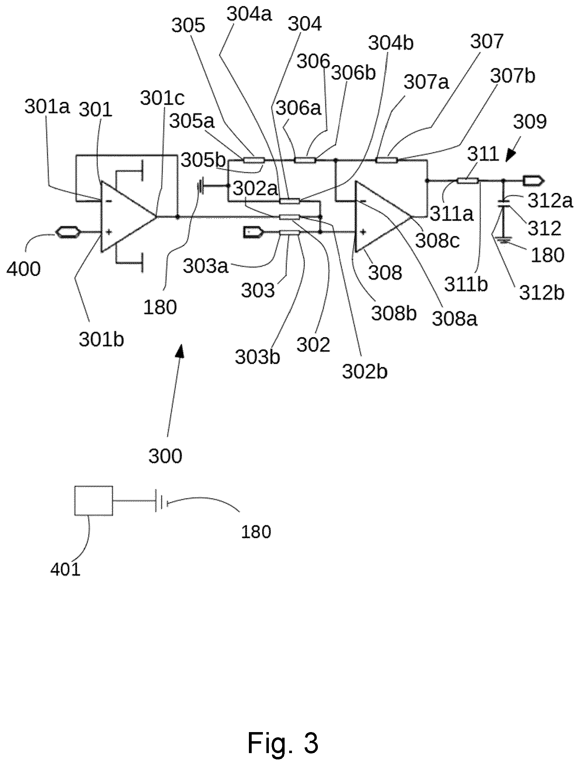

Fig. 3 illustrates an example of a potentiometric sensor circuit of a potentiometric sensor, according to an example. The potentiometric sensor is configured to measure the potential difference between a working electrode and a reference electrode forming part of such potentiometric sensor. The potentiometric sensor circuit 300 may comprise a voltage follower operational amplifier 301 including a negative input terminal 301a, a positive input terminal 301b and an output 301c, a resistor 302 including two terminals 302a, 302b, a resistor 303 including two terminals 303a, 303b, a resistor 304 including two terminals 304a, 304b, a resistor 305 including two terminals 305a, 305b, a resistor 306 including two terminals 306a, 306b, a resistor 307 including two terminals 307a, 307b and a summing amplifier 308. The summing amplifier 308 comprises a negative input terminal 308a, a positive input terminal 308b and an output 308c. The potentiometric sensor circuit further comprises a RC circuit 309.

The working electrode 400 of the potentiometric sensor may be coupled to the positive input terminal 301b of the voltage follower operational amplifier 301. The reference electrode 401 of the potentiometric circuit is coupled to ground 180 and it is further electrically connected to the working electrode of the amperometric sensor ( which is not shown in this figure but is shown in Fig. 2a), as hereinbefore described.

With such an arrangement, in use, an excitation voltage provided between the working electrode of the amperometric sensor and the reference electrode of the amperometric sensor, as hereinbefore described, is used as a reference voltage for the potentiometric sensor via the reference electrode 401 of the potentiometric sensor. The potentiometric sensor may thus measure the potential difference between a working electrode and a reference electrode wherein the reference electrode is provided with the potential difference between a working electrode and a reference electrode of the amperometric sensor. As a result, it is possible to perform measurements substantially simultaneously with the amperometric sensor and the potentiometric sensor without cross-talks or interferences.

Following the example, the voltage follower amplifier 301 is configured to convert the voltage sensed between the working electrode 400 and the reference electrode of the potentiometric sensor to a potentiometric proportional voltage.

The negative input terminal 301a of the follower amplifier may be coupled to the output 301c of such voltage follower operational amplifier. The output 301c may further be coupled to the terminal 302a of the resistor 302. The terminal 302b of the resistor 302 may further be coupled to the terminal 303b of the resistor 303, to the terminal 304b of the resistor 304 and to the positive input terminal 308b of the summing amplifier 308.

Moreover, the terminal 304a of the resistor 304 may be coupled to ground 180 and to the terminal 305a of the resistor 305. The terminal 305b of the resistor 305 may be coupled to terminal 306a of the resistor 306. The terminal 306b of the resistor 306 may also be coupled to negative input terminal 308a of the summing amplifier 308 and to the terminal 307a of the resistor 307. The terminal 307b of the resistor 307 may be coupled to the output 308c of the amplifier 308.

Similarly as in the amperometric circuit, the summing amplifier 308 may be configured to operate as an inverter amplifier if the proportional voltage provided to the summing amplifier is a negative voltage such that the negative voltage is converted to a positive voltage. In some other examples, if the proportional voltage outputted provided to the summing amplifier is a positive voltage, the summing amplifier 308 may be configured to operate as a follower amplifier. The reasoning of such an arrangement is that a control unit including an AC-DC converter (not shown in this figure) may be coupled to the output of the potentiometric circuit. The AC-DC converter may be configured to operate in a voltage range between 0 volts and 3.3 volts and thus, if a negative voltage is outputted by the operational amplifier, such negative voltage may not be detected by the AC-DC converter

The output 308c of the summing amplifier 308 may also be coupled to the RC circuit 309. The RC circuit comprises a resistor 311 including two terminals 311a, 311b, and a capacitor 312 including two terminals 312a, 312b.

The terminal 311a of the resistor 311 may be coupled to the output 308c of the amplifier 308. The terminal 311b of the resistor 311 may be coupled to the terminal 312a of the capacitor 312 and to the above-commented control module including an AC-DC converter (not shown in this figure), as will be explained later on. The terminal 312b of the capacitor may be coupled to ground 180.

As commented above, the reference electrode 401 of the potentiometric circuit is coupled to ground 180 and it is further electrically connected to the working electrode of the amperometric sensor, as hereinbefore described.

It is noted that an amperometric sensor and a potentiometric sensor as herein before described may be situated next to each other at a distance between 1 micrometre and 5 centimetres, optionally the amperometric and the potentiometric sensors are attached to a support substrate.

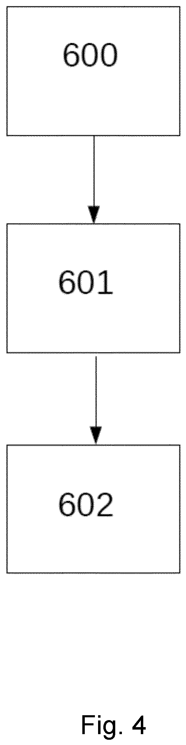

Fig. 4 illustrates a block diagram of a control module comprising an electrochemical sensor system, according to some examples.

The system may comprise an electromechanical sensor (array) system 600 as hereinbefore described, a reader module 601 e.g. a transceiver Huzzah ESP8266 and a control module 602.

The reader module 601 may be configured to receive a voltage from the amperometric sensor circuit of the amperometric sensor and a voltage from the potentiometric sensor circuit of the potentiometric. The voltages may be received substantially simultaneously. The reader module 601 may be provided e.g. with wireless functionality. With such an arrangement, the voltages may be wirelessly retrieved from the amperometric sensor and the potentiometric sensor, by the reader module 601, using standard operations.

In summary, the reader module 601 is configured to retrieve a voltage (signal) from the amperometric sensor circuit and a voltage (signal) from the potentiometric sensor circuit substantially simultaneously and provide such voltages to the control module 602 to be processed at the same time.

The control module 602 may be e.g. a PIC32MX795 microcontroller.

The control module 602 may comprise or may be implemented by electronic means, computing means or a combination of them, that is, said electronic or computing means may be used interchangeably so that a part of the described means may be electronic means and the other part may be computing means, or all described means may be electronic means or all described means may be computing means.

Examples of a control module 602 comprising only electronic means (that is, a purely electronic configuration) may be a programmable electronic device such as a CPLD (Complex Programmable Logic Device), an FPGA (Field Programmable Gate Array) or an ASIC (Application-Specific Integrated Circuit).

Examples of a control module 602 comprising only computing means may be a computer system (e.g. a laptop, a server, a desktop computer, an embedded or industrial computer, etc.), which may comprise a memory and a processor, the memory being adapted to store a set of computer program instructions, and the processor being adapted to execute these instructions stored in the memory in order to generate the various events and actions for which the control module 602 has been programmed.

The computer program may comprise program instructions for causing the control module 602 to perform a method for sensing analytical reactions and biological operations that will be described later on. The computer program may be embodied on a storage medium such as a ROM, for example a CD ROM or a semiconductor ROM, a magnetic recording medium, for example a hard disk, a solid-state disk (SSD), a USB flash drive (for example, a pen drive); or a non-volatile memory card such as a SD, miniSD or microSD card. In addition, the computer program may be carried on a transmissible carrier such as an electrical or optical signal, which may be conveyed via electrical or optical cable or by radio or other means.

When the computer program is embodied in a signal that may be conveyed directly by a cable or other device or means, the carrier may be constituted by such cable or other device or means.

Alternatively, the carrier may be an integrated circuit in which the computer program is embedded, the integrated circuit being adapted for performing, or for use in the performance of, the relevant methods.

The computer program may be in the form of source code, object code, a code intermediate source and object code such as in partially compiled form, or in any other form suitable for use in the implementation of the method. The carrier may be any entity or device capable of carrying the computer program.

Furthermore, the control module 602 may also have a hybrid configuration between computing and electronic means. In this case, the control module may comprise a memory and a processor to implement computationally part of its functionalities and certain electronic circuits to implement the remaining functionalities.

As commented above, in examples, the control module 602 may be a PIC32MX795 microcontroller. The microcontroller may comprise a 32 bits architecture comprising at least one USB port and at least one I2C port. The microcontroller 602 may be configured to receive at least one response voltage e.g. a voltage signal from the amperometric sensor circuit (see Fig. 2a) and the potentiometric sensor circuit (see Fig. 3). The voltage (signals) may be received substantially simultaneously i.e. (virtually) at the same time or with a difference of less than e.g. one second. In this respect, the microcontroller may be configured to: generate parameters related to the amperometric and potentiometric sensing (via the USB port), generate the power signals related to the amperometric measurements, perform substantially simultaneously the analogical-digital conversion related to the potentiometric and amperometric sensors, perform the selection of the gain resistor in the amperometric circuit (via the I2C port connected to the ADG728 as hereinbefore described) and transmit and receive data via the transceiver 601 e.g. a Huzzah WiFi ESP8266.

In any case, the control module 602 may be configured to execute a method for sensing analytical reactions and biological operations, wherein the method comprises:
- receiving the filtered offset potentiometric voltage from the potentiometric sensor circuit;
- receiving substantially simultaneously the output amperometric sensor voltage from the amperometric sensor circuit;
- determining substantially simultaneously a current measurement related to the received voltage from the amperometric sensor circuit and a voltage measurement related to the received voltage from the potentiometric sensor circuit.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

## Claims

1. An electrochemical sensor system for sensing analytical reactions and biological operations, wherein electrochemical sensor system comprises:
an amperometric sensor comprising two electrodes, wherein the electrodes comprise a working electrode (280) and a reference electrode, wherein the amperometric sensor includes no other electrodes;
a potentiometric sensor comprising two electrodes, wherein the electrodes comprise a working electrode (400) and a reference electrode (401), wherein the potentiometric sensor includes no other electrodes, wherein the reference electrode (401) of the potentiometric sensor is electrically coupled to ground, wherein the reference electrode (401) of the potentiometric sensor coupled to ground introduces a virtual zero in the working electrode (280) of the amperometric sensor;
wherein the working electrode (280) of the amperometric sensor is electrically coupled to the reference electrode (401) of the potentiometric sensor, and
wherein the amperometric sensor is configured to receive an excitation voltage between the working electrode (280) of the amperometric sensor and the reference electrode of the amperometric sensor such that the received excitation voltage is used as a reference voltage of the potentiometric sensor via the reference electrode of the potentiometric sensor.

2. The system according to claim 1, wherein the potentiometric sensor is an ion-selective sensor.

3. The system according to any one of claims 1-2, wherein the amperometric sensor and the potentiometric sensor are situated next to each other at a distance between 1 micrometre and 5 centimetres.

4. The system according to any one of claims 1-3, wherein the amperometric sensor is coupled to a pulse width modulator configured to provide the excitation voltage between the working electrode of the amperometric sensor and the reference electrode of the amperometric sensor.

5. The system according to claim 4, further comprising a low pass filter which is placed between the pulse width modulator circuit (100) and the amperometric sensor, wherein the low pass filter is configured to remove a noise voltage signal from the excitation voltage received by the pulse width modulator such that a noiseless excitation voltage is provided between the working electrode and the reference electrode of the amperometric sensor.

6. The system according to claim 5, wherein the low pass filter comprises a operational amplifier which is configured to operate either as an inverter amplifier for achieving a negative noiseless excitation voltage or as a voltage follower for achieving a positive noiseless excitation voltage.

7. The system according to any one of claims 1-6, wherein the amperometric sensor further comprises an amperometric sensor circuit (200) comprising a transimpedance amplifier (201), wherein the transimpedance amplifier comprises a positive input terminal (201a), a negative input terminal (201b) and an output terminal (201c), wherein the positive input terminal (201a) is connected to ground, wherein the negative input terminal (201b) is connected to the working electrode (280) of the amperometric sensor, wherein the transimpedance amplifier (201) is configured to convert an input current sensed via the working electrode (280) of the amperometric sensor to a proportional output voltage.

8. The system according to claim 7, wherein the transimpedance amplifier (201) further comprises a gain resistor (205) arranged between the negative input terminal (201a) of the transimpedance amplifier (201) and the output terminal (201c) of the transimpedance amplifier (201), wherein the amperometric sensor circuit further comprises a resistor gain circuit configured to select the gain resistor in a range between 1 Kohm to 33 Mohm.

9. The system according to any one of claims 7-8, wherein the amperometric sensor circuit further comprises an operational amplifier (206), wherein a positive input terminal (206b) and a negative input terminal (206a) of the operation amplifier (206) are coupled to the output terminal (201c) of the transimpedance amplifier (201), wherein the operational amplifier is configured to operate either as an inverter amplifier if the proportional voltage outputted by the transimpedance amplifier (201) is a negative voltage or as follower amplifier if the proportional voltage outputted by the transimpedance amplifier (TIA) is a positive voltage such that an output amperometric sensor voltage is obtained.

10. The system according to any one of claims 1-9, wherein the amperometric sensor further comprises a reference electrode circuit, wherein the reference electrode circuit comprises an operational amplifier (800), wherein the reference electrode of the amperometric sensor is coupled to a positive input terminal (800b) of the operational amplifier (800) of the reference electrode circuit.

11. The system according to any one of claims 1-10, wherein the potentiometric sensor further comprises a potentiometric sensor circuit (300) comprising a voltage follower amplifier (301), wherein the voltage follower amplifier (301) comprises a positive terminal (301b), a negative terminal (301a) and an output terminal (301c), wherein the positive terminal (301b) of the voltage follower amplifier (300) is coupled to the working electrode (400) of the potentiometric sensor, wherein the voltage follower amplifier is configured to convert the voltage sensed by the working electrode of the amperometric sensor to a proportional potentiometric voltage.

12. The system according to claim 11, wherein the potentiometric sensor circuit (300) further comprises a summing amplifier (308), wherein the summing amplifier (308) comprises an input positive terminal (308b), an input negative terminal (308a) and an output terminal (308c), wherein the output terminal (301c) of the voltage follower amplifier (301) of the potentiometric sensor is coupled to the input positive terminal (308b) and the input negative terminal (308a) of the summing amplifier (308), wherein the summing amplifier (308) is configured to apply an offset to the voltage received by the voltage follower amplifier (301) such that an offset potentiometric voltage is obtained.

13. The sensor system according to claim 11, wherein the potentiometric sensor circuit further comprises an RC circuit (309), wherein the RC circuit (309) is coupled to the output (308c) of the summing amplifier such that a filtered offset potentiometric voltage is obtained.

14. A control module (602) comprising:
at least one electrochemical sensor system according to any of claims 1-13;
a reader module (601) configured receive at least one response voltage from the potentiometric sensor circuit (300) comprised in the potentiometric sensor; and
at least one response voltage from the amperometric sensor circuit (200) comprised in the amperometric sensor;
the control module (602) being configured to:
- receive the filtered offset potentiometric voltage from the electrochemical potentiometric sensor circuit (300);
- receive substantially simultaneously the output amperometric voltage from the amperometric sensor circuit (200); and
- determine substantially simultaneously a current measurement related to the received output amperometric voltage from the amperometric sensor circuit and a voltage measurement related to the received filtered offset potentiometric voltage from the potentiometric sensor circuit (300).

15. A method for sensing analytical reactions and biological operations executed by the control module (602) according to claim 14, the method comprising:
- receiving the filtered offset potentiometric voltage from the potentiometric sensor circuit (300);
- receiving substantially simultaneously the output amperometric sensor voltage from the amperometric sensor circuit (200); and
- determining substantially simultaneously a current measurement related to the received output amperometric sensor voltage from the amperometric sensor circuit and a voltage measurement related to the received filtered offset potentiometric voltage from the potentiometric sensor circuit (300).

## Patentansprüche

1. Ein elektrochemisches Sensorsystem zum Erfassen analytischer Reaktionen und biologischer Vorgänge, wobei das elektrochemische Sensorsystem Folgendes umfasst:
einen amperometrischen Sensor, der zwei Elektroden umfasst, wobei die Elektroden eine Arbeitselektrode (280) und eine Referenzelektrode umfassen, wobei der amperometrische Sensor keine anderen Elektroden beinhaltet;
einen potentiometrischen Sensor, der zwei Elektroden umfasst, wobei die Elektroden eine Arbeitselektrode (400) und eine Referenzelektrode (401) umfassen, wobei der potentiometrische Sensor keine anderen Elektroden beinhaltet, wobei die Referenzelektrode (401) des potentiometrischen Sensors elektrisch an Masse gekoppelt ist, wobei die Referenzelektrode (401) des potentiometrischen Sensors, die an Masse gekoppelt ist, einen virtuellen Nullpunkt in die Arbeitselektrode (280) des amperometrischen Sensors einführt;
wobei die Arbeitselektrode (280) des amperometrischen Sensors elektrisch mit der Referenzelektrode (401) des potentiometrischen Sensors gekoppelt ist, und
wobei der amperometrische Sensor dazu konfiguriert ist, eine Anregungsspannung zwischen der Arbeitselektrode (280) des amperometrischen Sensors und der Referenzelektrode des amperometrischen Sensors zu empfangen, sodass die empfangene Anregungsspannung über die Referenzelektrode des potentiometrischen Sensors als Referenzspannung des potentiometrischen Sensors verwendet wird.

2. Das System nach Anspruch 1, wobei der potentiometrische Sensor ein ionenselektiver Sensor ist.

3. Das System nach einem der Ansprüche 1 bis 2, wobei der amperometrische Sensor und der potentiometrische Sensor in einem Abstand von zwischen 1 Mikrometer und 5 Zentimetern nebeneinander angeordnet sind.

4. Das System nach einem der Ansprüche 1 bis 3, wobei der amperometrische Sensor an einen Pulsweitenmodulator gekoppelt ist, der dazu konfiguriert ist, die Anregungsspannung zwischen der Arbeitselektrode des amperometrischen Sensors und der Referenzelektrode des amperometrischen Sensors bereitzustellen.

5. Das System nach Anspruch 4, ferner umfassend einen Tiefpassfilter, der zwischen der Pulsweitenmodulatorschaltung (100) und dem amperometrischen Sensor angeordnet ist, wobei der Tiefpassfilter dazu konfiguriert ist, ein Rauschspannungssignal von der durch den Pulsweitenmodulator empfangenen Anregungsspannung zu entfernen, so dass eine geräuschlose Anregungsspannung zwischen der Arbeitselektrode und der Referenzelektrode des amperometrischen Sensors bereitgestellt wird.

6. Das System nach Anspruch 5, wobei der Tiefpassfilter einen Operationsverstärker umfasst, der dazu konfiguriert ist, entweder als Inverterverstärker zum Erreichen einer negativen geräuschlosen Anregungsspannung oder als ein Spannungsfolger zum Erreichen einer positiven geräuschlosen Anregungsspannung zu arbeiten.

7. Das System nach einem der Ansprüche 1 bis 6, wobei der amperometrische Sensor ferner eine amperometrische Sensorschaltung (200) umfasst, die einen Transimpedanzverstärker (201) umfasst, wobei der Transimpedanzverstärker einen positiven Eingangsanschluss (201a), einen negativen Eingangsanschluss (201b) und einen Ausgangsanschluss (201c) umfasst, wobei der positive Eingangsanschluss (201a) an Masse verbunden ist, wobei der negative Eingangsanschluss (201b) mit der Arbeitselektrode (280) des amperometrischen Sensors verbunden ist, wobei der Transimpedanzverstärker (201) dazu konfiguriert ist, einen über die Arbeitselektrode (280) des amperometrischen Sensors erfassten Eingangsstrom in eine proportionale Ausgangsspannung umzuwandeln.

8. Das System nach Anspruch 7, wobei der Transimpedanzverstärker (201) ferner einen Verstärkungswiderstand (205) umfasst, der zwischen dem negativen Eingangsanschluss (201a) des Transimpedanzverstärkers (201) und dem Ausgangsanschluss (201c) des Transimpedanzverstärkers (201) angeordnet ist, wobei die amperometrische Sensorschaltung ferner eine Widerstandsverstärkungsschaltung umfasst, die dazu konfiguriert ist, den Verstärkungswiderstand in einem Bereich von zwischen 1 Kohm bis 33 Mohm auszuwählen.

9. Das System nach einem der Ansprüche 7 bis 8, wobei die amperometrische Sensorschaltung ferner einen Operationsverstärker (206) umfasst, wobei ein positiver Eingangsanschluss (206b) und ein negativer Eingangsanschluss (206a) des Operationsverstärkers (206) an den Ausgangsanschluss (201c) des Transimpedanzverstärkers (201) gekoppelt sind, wobei der Operationsverstärker dazu konfiguriert ist, entweder als Inverterverstärker zu arbeiten, wenn die von dem Transimpedanzverstärker (201) ausgegebene proportionale Spannung eine negative Spannung ist, oder als Folgeverstärker zu arbeiten, wenn die von dem Transimpedanzverstärker (TIA, englisch: *transimpedance amplifier)* ausgegebene proportionale Spannung eine positive Spannung ist, so dass eine amperometrische Sensorausgangsspannung erhalten wird.

10. Das System nach einem der Ansprüche 1 bis 9, wobei der amperometrische Sensor ferner eine Referenzelektrodenschaltung umfasst, wobei die Referenzelektrodenschaltung einen Operationsverstärker (800) umfasst, wobei die Referenzelektrode des amperometrischen Sensors mit einem positiven Eingangsanschluss (800b) des Operationsverstärkers (800) der Referenzelektrodenschaltung gekoppelt ist.

11. Das System nach einem der Ansprüche 1 bis 10, wobei der potentiometrische Sensor ferner eine potentiometrische Sensorschaltung (300) umfasst, die einen Spannungsfolgeverstärker (301) umfasst, wobei der Spannungsfolgeverstärker (301) einen positiven Anschluss (301b), einen negativen Anschluss (301a) und einen Ausgangsanschluss (301c) umfasst, wobei der positive Anschluss (301b) des Spannungsfolgeverstärkers (300) mit der Arbeitselektrode (400) des potentiometrischen Sensors gekoppelt ist, wobei der Spannungsfolgeverstärker dazu konfiguriert ist, die von der Arbeitselektrode des amperometrischen Sensors erfasste Spannung in eine proportionale potentiometrische Spannung umzuwandeln.

12. Das System nach Anspruch 11, wobei die potentiometrische Sensorschaltung (300) ferner einen Summierverstärker (308) umfasst, wobei der Summierverstärker (308) einen positiven Eingangsanschluss (308b), einen negativen Eingangsanschluss (308a) und einen Ausgangsanschluss (308c) umfasst, wobei der Ausgangsanschluss (301c) des Spannungsfolgeverstärkers (301) des potentiometrischen Sensors an den positiven Eingangsanschluss (308b) und den negativen Eingangsanschluss (308a) des Summierverstärkers (308) gekoppelt ist, wobei der Summierverstärker (308) dazu konfiguriert ist, einen Offset an die durch den Spannungsfolgeverstärker (301) empfangene Spannung anzulegen, sodass eine potentiometrische Offsetspannung erhalten wird.

13. Das Sensorsystem nach Anspruch 11, wobei die potentiometrische Sensorschaltung ferner ein RC-Glied (309) umfasst, wobei das RC-Glied (309) so an den Ausgang (308c) des Summierverstärkers gekoppelt ist, dass eine gefilterte potentiometrische Offsetspannung erhalten wird.

14. Ein Steuermodul (602), umfassend:
mindestens ein elektrochemisches Sensorsystem nach einem der Ansprüche 1 bis 13;
ein Lesemodul (601), das dazu konfiguriert ist, mindestens eine Ansprechspannung von der potentiometrischen Sensorschaltung (300), die in dem potentiometrischen Sensor enthalten ist; und
mindestens eine Ansprechspannung von der amperometrischen Sensorschaltung (200), die in dem amperometrischen Sensor enthalten ist, zu empfangen;
wobei das Steuermodul (602) dazu konfiguriert ist:
- die gefilterten potentiometrische Offsetspannung von der elektrochemischen potentiometrischen Sensorschaltung (300) zu empfangen;
- im Wesentlichen gleichzeitig die amperometrische Ausgangsspannung von der amperometrischen Sensorschaltung (200) zu empfangen; und
- im Wesentlichen gleichzeitig eine Strommessung in Bezug auf die empfangene amperometrische Ausgangsspannung von der amperometrischen Sensorschaltung und eine Spannungsmessung in Bezug auf die empfangene gefilterte potentiometrische Offsetspannung von der potentiometrischen Sensorschaltung (300) zu bestimmen.

15. Ein Verfahren zum Erfassen analytischer Reaktionen und biologischer Vorgänge, das durch das Steuermodul (602) nach Anspruch 14 ausgeführt wird, wobei das Verfahren Folgendes umfasst:
- empfangen der gefilterten potentiometrischen Offsetspannung von der potentiometrischen Sensorschaltung (300);
- empfangen im Wesentlichen gleichzeitig der amperometrischen Sensorausgangsspannung von der amperometrischen Sensorschaltung (200); und
- bestimmen im Wesentlichen gleichzeitig einer Strommessung in Bezug auf die empfangene amperometrische Ausgangssensorspannung von der amperometrischen Sensorschaltung und einer Spannungsmessung in Bezug auf die empfangene gefilterte potentiometrische Offsetspannung von der potentiometrischen Sensorschaltung (300).

## Revendications

1. Un système de capteur électrochimique pour détecter des réactions analytiques et des opérations biologiques, dans lequel le système de capteur électrochimique comprend :
un capteur ampérométrique comprenant deux électrodes, dans lequel les électrodes comprennent une électrode de travail (280) et une électrode de référence, dans lequel le capteur ampérométrique ne comprend aucune autre électrode ;
un capteur potentiométrique comprenant deux électrodes, dans lequel les électrodes comprennent une électrode de travail (400) et une électrode de référence (401), dans lequel le capteur potentiométrique ne comprend aucune autre électrode, dans lequel l'électrode de référence (401) du capteur potentiométrique est électriquement couplée à la masse, dans lequel l'électrode de référence (401) du capteur potentiométrique couplée à la masse introduit un zéro virtuel dans l'électrode de travail (280) du capteur ampérométrique ;
dans lequel l'électrode de travail (280) du capteur ampérométrique est couplée électriquement à l'électrode de référence (401) du capteur potentiométrique, et
dans lequel le capteur ampérométrique est configuré pour recevoir une tension d'excitation entre l'électrode de travail (280) du capteur ampérométrique et l'électrode de référence du capteur ampérométrique de telle sorte que la tension d'excitation reçue est utilisée comme tension de référence du capteur potentiométrique via l'électrode de référence du capteur potentiométrique.

2. Le système selon la revendication 1, dans lequel le capteur potentiométrique est un capteur sélectif d'ions.

3. Le système selon l'une quelconque des revendications 1 à 2, dans lequel le capteur ampérométrique et le capteur potentiométrique sont situés l'un à côté de l'autre à une distance comprise entre 1 micromètre et 5 centimètres.

4. Le système selon l'une quelconque des revendications 1 à 3, dans lequel le capteur ampérométrique est couplé à un modulateur de largeur d'impulsion configuré pour fournir la tension d'excitation entre l'électrode de travail du capteur ampérométrique et l'électrode de référence du capteur ampérométrique.

5. Le système selon la revendication 4, comprenant en outre un filtre passe-bas qui est placé entre le circuit modulateur de largeur d'impulsion (100) et le capteur ampérométrique, dans lequel le filtre passe-bas est configuré pour supprimer un signal de tension de bruit de la tension d'excitation reçue par le modulateur de largeur d'impulsion de sorte qu'une tension d'excitation sans bruit est fournie entre l'électrode de travail et l'électrode de référence du capteur ampérométrique.

6. Le système selon la revendication 5, dans lequel le filtre passe-bas comprend un amplificateur opérationnel qui est configuré pour fonctionner soit comme un amplificateur inverseur pour obtenir une tension d'excitation sans bruit négative, soit comme un suiveur de tension pour obtenir une tension d'excitation sans bruit positive.

7. Le système selon l'une quelconque des revendications 1 à 6, dans lequel le capteur ampérométrique comprend en outre un circuit de capteur ampérométrique (200) comprenant un amplificateur à transimpédance (201), dans lequel l'amplificateur à transimpédance comprend une borne d'entrée positive (201a), une borne d'entrée négative (201b) et une borne de sortie (201c), dans lequel la borne d'entrée positive (201a) est connectée à la masse, dans lequel la borne d'entrée négative (201b) est connectée à l'électrode de travail (280) du capteur ampérométrique, dans lequel l'amplificateur à transimpédance (201) est configuré pour convertir un courant d'entrée détecté via l'électrode de travail (280) du capteur ampérométrique en une tension de sortie proportionnelle.

8. Le système selon la revendication 7, dans lequel l'amplificateur à transimpédance (201) comprend en outre une résistance de gain (205) agencée entre la borne d'entrée négative (201a) de l'amplificateur à transimpédance (201) et la borne de sortie (201c) de l'amplificateur à transimpédance (201), dans lequel le circuit de capteur ampérométrique comprend en outre un circuit de gain de résistance configuré pour sélectionner la résistance de gain dans une plage comprise entre 1 Kohm et 33 Mohm.

9. Le système selon l'une quelconque des revendications 7 à 8, dans lequel le circuit de capteur ampérométrique comprend en outre un amplificateur opérationnel (206), dans lequel une borne d'entrée positive (206b) et une borne d'entrée négative (206a) de l'amplificateur opérationnel (206) sont couplées à la borne de sortie (201c) de l'amplificateur à transimpédance (201), dans lequel l'amplificateur opérationnel est configuré pour fonctionner soit comme un amplificateur inverseur si la tension proportionnelle délivrée par l'amplificateur à transimpédance (201) est une tension négative, soit comme un amplificateur suiveur si la tension proportionnelle délivrée par l'amplificateur à transimpédance (TIA, anglais : *transimpedance amplifier*) est une tension positive de sorte qu'une tension de capteur ampérométrique de sortie est obtenue.

10. Le système selon l'une quelconque des revendications 1 à 9, dans lequel le capteur ampérométrique comprend en outre un circuit d'électrode de référence, dans lequel le circuit d'électrode de référence comprend un amplificateur opérationnel (800), dans lequel l'électrode de référence du capteur ampérométrique est couplée à une borne d'entrée positive (800b) de l'amplificateur opérationnel (800) du circuit d'électrode de référence.

11. Le système selon l'une quelconque des revendications 1 à 10, dans lequel le capteur potentiométrique comprend en outre un circuit de capteur potentiométrique (300) comprenant un amplificateur suiveur de tension (301), dans lequel l'amplificateur suiveur de tension (301) comprend une borne positive (301b), une borne négative (301a) et une borne de sortie (301c), dans lequel la borne positive (301b) de l'amplificateur suiveur de tension (300) est couplée à l'électrode de travail (400) du capteur potentiométrique, dans lequel l'amplificateur suiveur de tension est configuré pour convertir la tension détectée par l'électrode de travail du capteur ampérométrique en une tension potentiométrique proportionnelle.

12. Le système selon la revendication 11, dans lequel le circuit de capteur potentiométrique (300) comprend en outre un amplificateur de sommation (308), dans lequel l'amplificateur de sommation (308) comprend une borne positive d'entrée (308b), une borne négative d'entrée (308a) et une borne de sortie (308c), dans lequel la borne de sortie (301c) de l'amplificateur suiveur de tension (301) du capteur potentiométrique est couplée à la borne positive d'entrée (308b) et à la borne négative d'entrée (308a) de l'amplificateur de sommation (308), dans lequel l'amplificateur de sommation (308) est configuré pour appliquer un offset à la tension reçue par l'amplificateur suiveur de tension (301) de sorte qu'une tension potentiométrique offset est obtenue.

13. Le système de capteur selon la revendication 11, dans lequel le circuit de capteur potentiométrique comprend en outre un circuit RC (309), dans lequel le circuit RC (309) est couplé à la sortie (308c) de l'amplificateur de sommation de sorte qu'une tension potentiométrique offset filtrée est obtenue.

14. Un module de commande (602) comprenant :
au moins un système de capteur électrochimique selon l'une quelconque des revendications 1 à 13 ;
un module de lecteur (601) configuré pour recevoir au moins une tension de réponse provenant du circuit de capteur potentiométrique (300) compris dans le capteur potentiométrique ; et
au moins une tension de réponse provenant du circuit de capteur ampérométrique (200) compris dans le capteur ampérométrique ;
le module de commande (602) étant configuré pour :
- recevoir la tension potentiométrique offset filtrée provenant du circuit de capteur potentiométrique électrochimique (300) ;
- recevoir essentiellement simultanément la tension ampérométrique de sortie provenant du circuit de capteur ampérométrique (200) ; et
- déterminer essentiellement simultanément une mesure de courant liée à la tension ampérométrique de sortie reçue provenant du circuit de capteur ampérométrique et une mesure de tension liée à la tension potentiométrique offset filtrée reçue provenant du circuit de capteur potentiométrique (300).

15. Un procédé de détection de réactions analytiques et d'opérations biologiques exécutées par le module de commande (602) selon la revendication 14, le procédé comprenant:
- recevoir la tension potentiométrique offset filtrée provenant du circuit de capteur potentiométrique (300) ;
- recevoir essentiellement simultanément la tension de capteur ampérométrique de sortie provenant du circuit de capteur ampérométrique (200) ; et
- déterminer essentiellement simultanément une mesure de courant liée à la tension de capteur ampérométrique de sortie reçue provenant du circuit de capteur ampérométrique et une mesure de tension liée à la tension potentiométrique offset filtrée reçue provenant du circuit de capteur potentiométrique (300).
